# EUROPEAN PATENT APPLICATION

(11) **EP 1 547 540 A1**
(43) Date of publication of application: **29.06.2005**
(21) Application number: 03027502.8
(22) Date of filing: 28.11.2003
(51) Int. Cl.: A61B 19/00

(54) **Apparatus for directing a magnetic element in a body of a patient**

(71) Applicant: SIEMENS AKTIENGESELLSCHAFT, 80333 München (DE); Stereotaxis Inc., St. Louis, MO 63108 (US)
(72) Inventor: Hambüchen, Klaus, 91334 Hemhofen (DE); Hogg, Bevil, 95060 Santa Cruz CA (US); Killmann, Reinmar, Dr., 91301 Forchheim (DE); Meyer, Andreas, 91096 Möhrendorf (DE); Nekovar, Anton, 91077 Neunkirchen (DE)
(74) Representative: Berg, Peter, Dipl.-Ing.

(57) **Abstract**

An Apparatus for directing a magnetic element in a body (17) of a patient comprises a pair of directing magnet pods (11, 12) movable in relation to each other and attached to opposite ends (10) of an arm (2, 20) which extends in a circumferential direction (4, 22) around a support (5) of the patient and which is movable in a circumferential direction (4, 22) and pivotable around a radial axis (8, 24) with respect to the support of the patient.

## Description

The present invention relates to an apparatus for directing a magnetic element in a body of a patient comprising a directing magnet arranged next to a support of the patient and movable with respect to the patient.

An apparatus of this type is known from US 6,459,924 Bl. The known apparatus comprises a strong permanent magnet or a cored solenoid mounted on an articulation support. The articulation support is provided with control mechanisms in order to enable movement of the permanent magnet or the cored solenoid along an arcuate arm as well as in a radial direction. Motion of the entire arm may optionally be provided by pivoting the arcuate arm around an axis extending in a radial direction.

Due to the simple geometry of the magnetic field, the known apparatus can be easily controlled. If a magnetic field of a certain strength is required within the body of a patient for directing a magnetic element, for example a magnetic seed or tip of a catheter equipped with a permanent magnet, the permanent magnet or the cored solenoid is moved until a magnet field with the desired strength and direction is present at the given point.

The known apparatus has the disadvantage that the geometry of the magnetic field itself may not be varied. Only the strength of the magnetic field generated by an electric current may be scaled by adjusting the strength of an electric current, but the spatial relations remain the same. Therefore, it is quite possible to provide in the known apparatus a magnetic field with a desired strength and direction as well as a desired field gradient by adjusting the current in the solenoid or by suitably moving the magnet, but the process of adjusting the magnet field can be quite time consuming under certain circumstances. Furthermore, there might be situations where there is a need to maintain a minimum field strength during the orientation process of the magnetic field, for example if the magnetic element is moved in blood vessels since the blood current in the vessels may exert some force on the magnetic element. In order to hold the magnetic element in place these forces must be compensated by corresponding magnetic forces.

From US 6,148,823 is known another apparatus for guiding a magnetic element within a selected region of a patient's body. The known apparatus comprises a toroid forming a magnetic circuit. Within the toroid a gap is formed for allowing a selected region of the patient's body to be positioned within the toroid. The magnetic flux is created by two permanent magnets arranged at both sides of the gap. Furthermore a magnetic return path made from an inexpensive permeable magnetic material connects both permanent magnets. The whole magnetic assembly is mounted on a movable magnet support which allows the magnet assembly to be moved with respect to the patient to provide the necessary freedom in establishing a magnetic field in a particular direction in a patient.

These known apparatus can for example be used to guide a magnet tip on a catheter as described in US 6,562,019. This type of catheter is generally used for guided myocardial treatments. Furthermore, these known apparatus can also be used for guiding a magnet tip on a guide wire as disclosed in US 6,428,551. This type of guide wire and magnet tip is generally be used for removing material from body lumens.

Although the known apparatus shows a high degree of flexibility in orienting the magnetic field as a whole the geometry of the magnetic field itself cannot easily be shaped which implies the same disadvantages as mentioned above.

Starting from this related art, the present invention is based on the object of providing an apparatus for guiding a magnetic element in a body of a patient with improved flexibility.

According to the present invention this object is achieved by an apparatus with the features of the independent claim. Further advantageous embodiments and refinements are subject matter of the dependent claims.

The apparatus for guiding a magnetic element in a patient's body comprises a pair of directing magnet pods producing a common magnetic field extending to the patient. For the purpose of this application the term "magnet pod" refers to a unit comprising a magnet and an articulating apparatus and sometimes a cover. The common magnetic field can be varied by changing at least one of the magnetic fields generated by one of the directing magnet pods. Furthermore, both directing magnet pods are attached to a support device by which the arrangement of the directing magnets is movable in relation to the support of the patient as an integral assembly.

The apparatus according to the invention allows choosing the shape and the orientation of the magnetic field independently. The shape of the magnetic field is chosen by changing the spatial relationship of the directing magnet pods, as the resulting magnetic field in the space between the two magnets results from the superposition of the magnetic fields generated by each of the directing magnet pods separately. The shape or geometry of the magnetic field in the space between both magnets therefore depends on the orientation and the spatial relationship of one magnet pod to the other. Accordingly a required strength, gradient or orientation of the magnetic field can easily be provided at nearly any point in the patient's body. In particular the direction of a magnetic force or torque acting on the magnetic element can easily be changed by moving the arrangement of both directing magnet pods as a whole by actuating the support device. In consequence the apparatus can be operated with a high degree of flexibility. This flexibility is achieved even if permanent magnets are used.

In one preferred embodiment where the directing magnet pods include permanent magnets the directing magnet pods can be moved in relation to each other in order to vary the common magnetic field.

In another preferred embodiment the directing magnet pods are movably mounted on opposite ends of an arm which extends in a circumferential direction around the support of the patient and which is movable in circumferential direction and pivotable around a radial axis with respect to the patient's support. By this design the magnet pods themselves can be brought at nearly any point of a sphere surrounding the patient's support. Furthermore the magnet pods can be moved in relation to each other. The shape of the magnetic field and the orientation of the magnetic field can therefore be changed separately.

By arranging both magnet pods on opposite ends of an arcuate arm, the torque applied to a bearing which holds the arm is minimized. In consequence, even heavy magnet pods with a weight in the range above 70 kg can be attached to the ends of the arcuate arm.

In another preferred embodiment, the bearing for the arcuate arm can be attached to the ceiling of a room housing the apparatus. In such an arrangement, the arcuate arm can be moved without engaging further medical equipment which might be positioned in the vicinity of the patient's support. Furthermore this arrangement allows any positioning of a combined x-ray imaging system over a range of imaging angles of up to at least +/- 60 degrees from vertical, permitting the user to perform magnetic navigation over the complete range of imaging angles.

Further features and advantages of the present invention become apparent by means of the following description when taken in conjunction with the accompanying drawing.
- Figure 1: shows a front view of one embodiment of the apparatus according to the invention.
- Figure 2: shows a side view of a second embodiment of the invention.
- Figure 3: shows a perspective view of the embodiment according to figure 1 complemented by an additional imaging system.
- Figure 4: shows a view from above of a third embodiment which comprises a arcuate support device and an imaging arm
- Figure 5: shows a perspective view of the third embodiment of figure 4 after the imaging arm has been pivoted by a relatively small angle of rotation.
- Figure 6: shows the embodiment according to figure 4 and 5 after the imaging arm have been pivoted by an large angle of rotation.

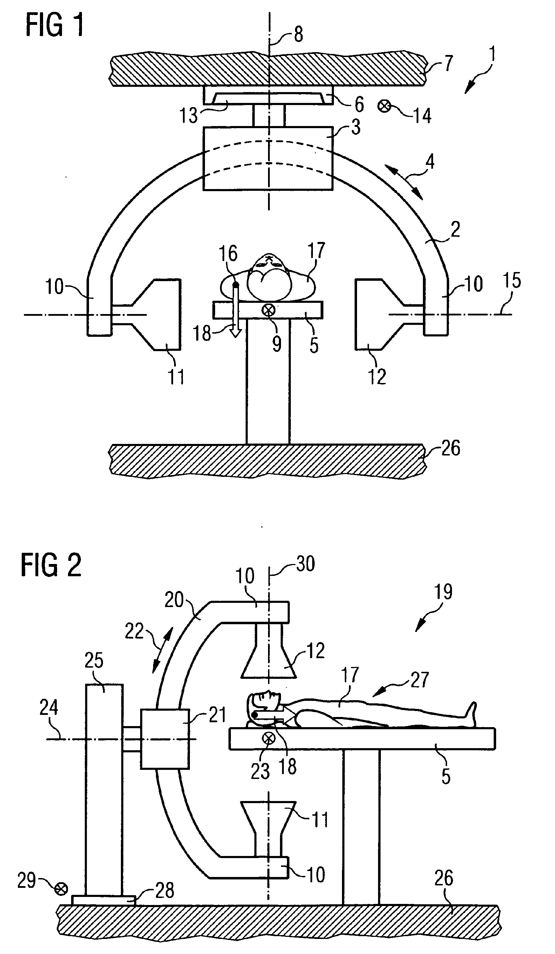

Figure 1 shows an apparatus 1 for directing a magnetic element in a body of a patient. For purposes of describing the present invention the term "directing" may include either or both of guiding and moving such as by pushing or pulling.

Apparatus 1 comprises an arcuate support device 2 which is held by a bearing 3. The bearing 3 houses a driving mechanism which allows the arcuate support device 2 to be moved in a circumferential direction 4 around a bed 5 on which a patient to be treated can be laid upon.

The bearing 3 is pivotably attached to a holder 6 which is fixed to a ceiling 7 of a room housing the apparatus 1. By a suitable driving means arcuate support device 2 can be pivoted around a radial axis 8 extending in a radial direction with respect to arcuate support device 2. Therefore, arcuate support device 2 can be pivoted around radial axis 8 and by the movement in circumferential direction 4 around an additional rotation axis 9 usually located in bed 5 or in the vicinity thereof and oriented orthogonally with respect to the plane extended by arcuate support device 2.

At opposite ends 10 of arcuate support device 2 directing magnet pods 11 and 12 are provided. These magnet pods 11 and 12 show a multipolar magnetic structure. The magnet pods 11 and 12 preferably contain permanent magnetic heads but may also contain electromagnetic devices. The weight of each magnet pod 11 or 12 may be in the range above 70 kg. The whole weight which arcuate support device 2 has to carry may amount up to 700 kg.

The holder 6 to which arcuate support device 2 is attached may comprise a rail system 13 extending in a longitudinal direction along the ceiling and allowing arcuate support device 2 to perform a translational displacement 14.

Furthermore magnet pods 11 and 12 can also be moved closer or further away from the body 17 along translation axis 15.

By the magnet pods 11 and 12 a magnetic field is created which extends in the space between the two magnet pods 11 and 12. At a given point 16 within a body 17 of a patient positioned in the space between the magnet pods 11 and 12 the magnetic field has a strength and an orientation which results from the addition of the two magnetic fields created by the magnet pods 11 and 12 respectively. In figure 1 the magnetic field at point 16 is represented by a magnetic field vector 18. By swiveling the magnetic heads inside magnet pods 11 and 12 around at least one of the orthogonal rotation axes intersecting the magnet pods 11 or 12 a certain field strength, orientation and gradient can be created at point 16 within the body 17 of the patient. For example a strong gradient may be provided by swiveling both magnetic heads inside magnet pods 11 and 12 towards the ceiling 7. If rotation axis 9 coincides with point 16 or lies in the vicinity of point 16 the orientation of the magnetic field vector 18 can be changed without altering the length of the magnetic field vector 18 simply by rotating arcuate support device 2 around rotation axis 9. In apparatus 1 it is thus possible to decouple the orientation of magnetic forces or torques from their amount. Both can be chosen independently according to the demands for moving the magnetic element in body 17. Apparatus 1 therefore provides a high degree of flexibility in moving the magnetic element in body 17. This is especially advantageous if the magnetic element must be directed in blood vessels where pressure forces resulting from the blood current in the vessel are acting on the magnetic element.

If the magnet pods 11 and 12 contain electromagnetic devices the common magnetic field between both magnet pods 11 and 12 can also be changed by adjusting the current of one of the electromagnetic devices inside magnet pod 11 or 12.

It should be appreciated that the magnet pods 11 and 12 need not necessarily be arranged along a common longitudinal axis on opposite sides of the bed 5. For example the angular distance between the magnet pods 11 and 12 with respect to the rotation axis 9 may also be 90° or 120° degrees. In some instances there might be also a driving mechanism which moves the magnet pods 11 and 12 along arcuate support device 2 changing the angular distance between them. In these cases the body 17 of the patient should still be positioned between the magnet pods 11 and 12 in order to ensure sufficient magnetic field strength.

Figure 2 shows another apparatus 19 comprising an arcuate support device 20 mounted in a bearing 21 and movable in a circumferential direction 22 around bed 5 which supports body 17 of a patient. By moving arcuate support device 20 in circumferential direction 22 arcuate support device 20 is effectively moved around a rotation axis 23 located in the bed 5 or in the patient's body 17. Furthermore arcuate support device 20 can be rotated around a horizontal rotation axis 24 extending through a stand 25 holding bearing 21. Stand 25 is mounted on floor 26 of a room housing apparatus 19. Magnet pods 11 and 12 are mounted at the end 10 of arcuate support device 20. In the embodiment shown in figure 2 arcuate support device 20 is able to perform a rotation around 360 degrees around patient's body 17.

In the embodiment shown in figure 2 a abdominal region 27 of body 27 can not easily be reached. For this and other reasons stand 25 may also be mounted on a rail system 28 for a translational displacement 29 of stand 25. Thus stand 25 can be brought in a more favorite position for treating abdominal region 27.

As in figure 1 magnet pods 11 and 12 can be moved closer or further away from the body 17 along a translation axis 30.

It should be appreciated that apparatus 1 and apparatus 19 may also be combined in a common apparatus which includes arcuate support device 2 and arcuate support device 20 and where the magnet pods 11 and 12 are activated as needed.

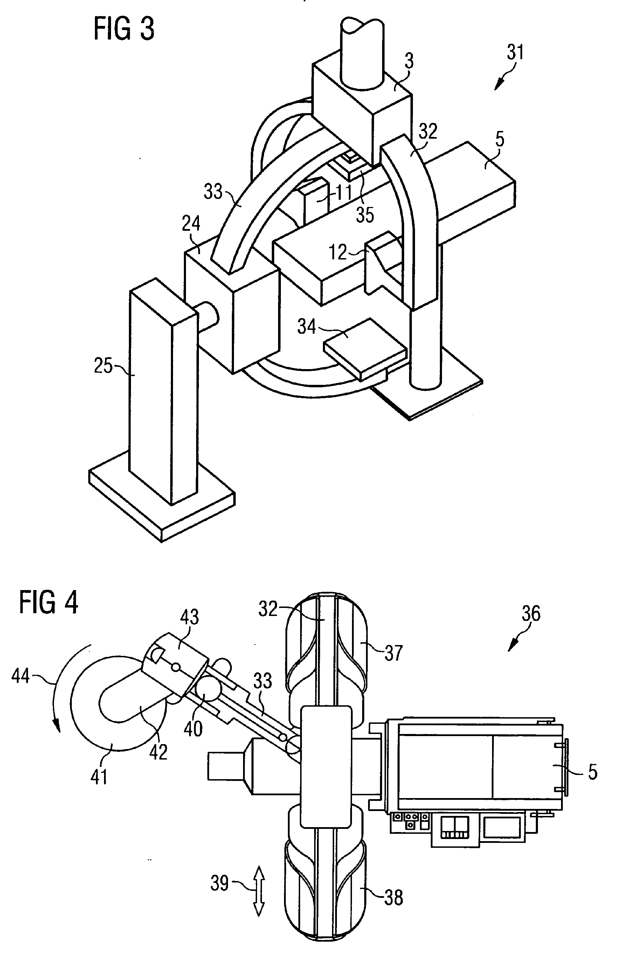

Furthermore, it should be noted that apparatus 1 may be complemented by an arcuate support device 20 of the type shown in figure 2. This results in an apparatus 31 as shown in figure 3. Apparatus 31 comprises an arcuate support device 32 which carries magnet pods 11 and 12. Apparatus 31 further includes an imaging arm 33 with an x-ray source 34 and an x-ray detector 35 for monitoring the movement of the magnetic element in body 17. This arrangement allows any positioning of the combined x-ray imaging system over a range of imaging angles of more than +/- 30 degrees, preferably more than +/- 60 degrees from vertical, permitting the user to perform magnetic navigation over the complete range of imaging angles.

Also apparatus 19 may be complemented by an arcuate support device 2 of the type shown in figure 1 and equipped with imaging devices for monitoring the movement of the magnetic element. In this case the imaging system may positioned over a range of imaging angles of more than +/- 30 degrees, preferably more than +/- 60 degrees from horizontal.

Figure 4 shows a view from above of an apparatus 33 which constitute a modified embodiment with respect with the embodiment shown in figure 3. Apparatus 36 has cylindrical magnet pods 37 and 38 attached to arcuate support device 32 as in the embodiments according to figure 1 and 3. Magnet pods 37 and 38 may include electromagnetic devices or permanent magnets. Furthermore magnet pods 37 and 38 may perform a translational movement in a longitudinal direction 39.

In apparatus 36 imaging arm 33 is mounted in a bearing 40 attached to a base 41 by an arm 42 extending in a horizontal direction and by an upright stand 43. The base 41 is positioned off-axis with respect to bed 5. Arm 42 is pivotably connected to base 41 and must be pivoted in a rotation direction 44 if the imaging arm 30 has to be aligned with the longitudinal axis of bed 5.

This arrangement allows the isocentre of arcuate support device 32 and the isocentre of imaging arm 33 to be moved in the abdominal region of body 17. Because of the off-axis position of stand 41 bed 5 can be shifted in the longitudinal direction so that the whole abdominal region of body 17 is translated in the common isocentre of arcuate support device 32 and imaging arm 33.

It should be noted that arcuate support device 32 can be moved toward the feet of body 17 if imaging arm 33 is in the off-axis position depicted in figure 4 since the isocentre of imaging arm 33 is moved toward the feet of body 17 if imaging arm 33 is brought in an off-axis position.

The movement of arcuate support device 32 is realized by means of a rail system attached to the ceiling and extending along bed 5. Advantageously the rail system should extend so far in longitudinal direction that arcuate support device 32 might be moved behind imaging arm 33. This allows apparatus 36 to be used for imaging purposes without magnetic navigation. Furthermore, the positioning of the patient to be treated can be performed more easily.

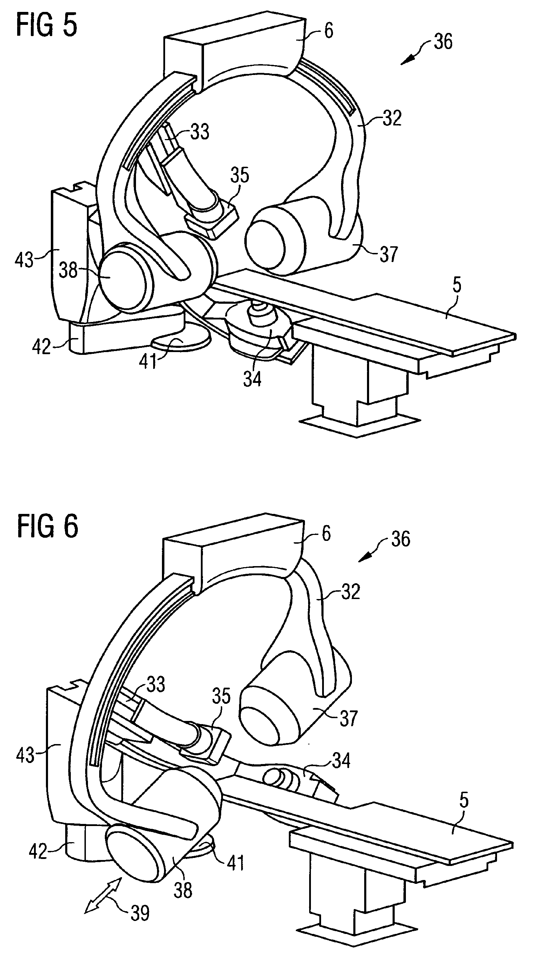

Figures 5 and 6 finally show apparatus 36 during operation in a perspective view. In figure 5 imaging arm 33 has performed an angular movement around a rotation axis which extends along the longitudinal direction of bed 5 and corresponds to the rotation axis 24 as in figure 2. As depicted in figure 5 the angular movement of imaging arm 33 must be stopped since x-ray detector 35 is about to get into contact with magnet pod 38. However, as shown in figure 6 the angular movement of imaging arm 33 might be resumed if arcuate support device 32 performs an angular movement in the same direction. As already mentioned above apparatus 36 and apparatus 31 allow any positioning of imaging arm 33 over a range of imaging angles of more than +/- 60° degrees from vertical.

Advantageously imaging arm 33 and arcuate support device 32 are moved synchronously. In particular the magnetic devices inside magnet pods 37 and 38 are guided such, that the orientation and strength of the magnetic field in the isocentre of arcuate support device 32 remains essentially unchanged during the movement of arcuate support device 32.

It is also possible to wait with the angular movement of arcuate support device 32 until imaging arm 33 nearly hits one of the magnet pods 37 or 38. The arcuate support device 32 must then be moved if the angular movement of imaging arm 33 should be continued.

The magnet pods 37 and 38 may be provided with a collision detection system which might be used as auxiliary means for guiding the rotational movement of the arcuate support device 32 and the imaging arm 33 around bed 5 and the patient deposited thereon. If one of the magnet pods 37 and 38 is in danger of colliding with the patient the event will be detected by the collision detection system. Then the colliding magnet pod 37 or 38 can be retracted in longitudinal direction 39 and the angular movement can be resumed until the desired angular position of the imaging arm 33 is reached. If the desired angular position has been reached, the magnet pod 37 or 38 which has been retracted can be advanced until the distance between the magnet pods 37 and 38 has been reduced to the amount necessary for the intended magnetic navigation.

The apparatus 1, 19, 31 and 36 are especially adapted for interventional procedures in the angiographic and cardangiographic medical field. For this purpose it is necessary to navigate and move catheters and guide wires within the heart. For navigation with magnetic fields, these are equipped with a small permanent magnet in their tips. Particularly coronary vessel diseases and cardiac arrhythmias can be treated by using apparatus 1, 19, 31 and 36.

A treatment of coronary vessel diseases involves navigating a guide wire to the target point in the diseased vessel, advancing a catheter over the guide wire to the target point and performing the desired treatment, for example a balloon angioplasty or a stent placement.

Cardiac arrhythmias are often treated by so-called ablation procedures, wherein a catheter is advanced into the cardiac chambers via veins or arteries. In the cardiac chambers, the tissue causing the arrhythmias is ablated, thus leaving the previously arrhythmogenic substrate as necrotic tissue. In general angiographic and neuroradiographic procedures are conducted in a similar way, treating aneurysms, atriovenous malformations and alike in a minimally invasive way.

By the apparatus 1, 19, 31 and 36 the movement of the guide wire can be controlled by means of the magnetic field vector 18. The various embodiments as described in this application enable remote control of catheters and guide wires equipped with a magnetic tip.

Since the x-ray image normally used for observing the movement of the catheter or guide wire is displayed in the control room of a catheterization laboratory, remote control of the device from the control room is possible. A remote control of the movement, however, is a big advantage for the physician as the exposure of the physician to radiation is significantly reduced.

## Claims

**1.** Apparatus for directing a magnetic element in a body (17) of a patient comprising a directing magnet pods arranged next to a support (5) of the patient (17) and movable with respect to the patient (17),
**characterized in that**
the apparatus comprises an arcuate support device (2, 20, 32) providing movement with at least two degrees of freedom (8, 9, 14, 15, 23, 24, 29, 30, 39), a pair of directing magnet pods (11, 12, 37, 38) producing a changeable common magnetic field extending to the patient (17) which are both attached to the support device (2, 20, 32) by which the arrangement of the directing magnet pods (11, 12, 37, 38) is movable in relation to the support (5) of the patient (17) as an integral assembly.

**2.** Apparatus according to claim 1,
**characterized in that**
the directing magnet pods (11, 12, 37, 38) are attached to opposite ends (10) of an arm (2, 20, 32) which extends in a circumferential direction (4, 22) around the support (5) of the patient (17) and which is movable in a circumferential direction (4, 22) and pivotable around a radial axis (8, 24) with respect to the support (5) of the patient.

**3.** Apparatus according to one of the claims 1 or 2,
**characterized in that**
the support device comprises an arcuate arm (2, 20, 32) slidably mounted in a bearing.

**4.** Apparatus according to one of the claims 1 to 3,
**characterized in that**
the support device (2, 20, 32) is located above the support (5) of the patient (17).

**5.** Apparatus according to claim 4,
**characterized in that**
the arm (2, 32) is held by a bearing (3) located above the support (5) of the patient (17).

**6.** Apparatus according to claim 5,
**characterized in that**
the ends of the arm (2, 32) are movable more than +/- 30° with respect to a horizontal plane.

**7.** Apparatus according to one of the claims 1 to 3,
**characterized in that**
the support device (20) is located besides the support (5) of the patient (17).

**8.** Apparatus according to claim 1,
**characterized in that**
the apparatus comprises a pair of directing magnet pods (11, 12, 37, 38) movable in relation to each other.

**9.** Apparatus according to claim 2 or 3,
**characterized in that**
the pair of directing magnet pods (11, 12, 37, 38) is pivotably mounted on opposite ends (10) of the support device (2, 20, 32).

**10.** Apparatus according to one of the claims 1 to 9,
**characterized in that**
the arcuate support device (2, 20, 32) is combined with an arcuate arm (33) for an imaging system (34, 35).

**11.** Apparatus according to claim 10,
**characterized in that**
the support device (2, 20, 32) for the magnet pods (11, 12, 37, 38) is mounted above the support (5) of the patient (17) and the arm (33) for the imaging system (34, 35) is mounted besides the support (5) of the patient (17).

**12.** Apparatus according to claim 11,
**characterized in that**
the arm (33) for the imaging system (34, 35) is attached to a holder (41, 42, 43) which allows the arm (33) for the imaging system (34, 35) to be located in an off-axis position with respect to the longitudinal axis of the support (5) of the patient (17).

**13.** Apparatus according to claim 12,
**characterized in that**
the holder (41, 42, 43) moves the arm (33) for the imaging system (34, 35) towards the foot end of the support (5) of the patient (17) in the off-axis position.

**14.** Apparatus according to one of the claims 10 or 13,
**characterized in that**
the movement of the support device (2, 20, 32) for the magnet pods(11, 12, 37, 38) and the movement of the support device (33) for the imaging system (34, 35) is performed taking into account the relative position of both support devices with respect to each other.

**15.** Apparatus according to one of the claims 1 to 14,
**characterized in that**
the magnet pods (11, 12, 37, 38) are movable around two rotation axes (8, 9, 23, 24) and along two translation axes (14, 15, 29, 30, 39).

**16.** Method for treating a patient in the abdominal region,
**characterized in that**
an apparatus according to one of the claims 1 to 15 is used.

**17.** Method according to claim 16,
**characterized in that**
the patient is treated in the region of the liver.

**17.** Method according to claim 16,
**characterized in that**
the patient is treated in the urogenital region.

**18.** Method according to claim 16,
**characterized in that**
the patient is treated in the region of a leg.
